# EUROPEAN PATENT APPLICATION

(11) **EP 1 808 479 A1**
(43) Date of publication of application: **18.07.2007**
(21) Application number: 05027588.2
(22) Date of filing: 16.12.2005
(51) Int. Cl.: C11D 3/40

(54) **Personal care composition**

(71) Applicant: Cognis IP Management GmbH, 40589 Düsseldorf (DE)
(72) Inventor: Asensio, Juan-Antonio, Dr., 08820 El Prat de Llobregat Barcelona (ES); Viladot Petit, Josep-Lluis, Dr., 08018 Barcelona (ES); Gomez, Yolanda, 08170 Montornés del Vallés Barcelona (ES); De Moragas, Maria, Dr., 08310 Argentona Barcelona (ES)
(74) Representative: Fabry, Bernd

(57) **Abstract**

**Summary**

Suggested are personal care compositions comprising encapsulated dyes.

## Description

### Object of the invention

The present invention relates to personal care compositions, more particularly to liquid soaps and hand washing pastes comprising encapsulated dyes.

### State of the art

Personal cleansing compositions are an important part of the daily hygienic regimen for a large group of both women and men throughout the world. Particularly hand cleansing products have traditionally been marketed in a variety of forms such as bar soaps, creams, lotions, pastes and gels. The amount of time needed to clean the skin or a surface has been researched extensively. The Association for Professionals in Infection Control and Epidemiology (APIC) Guideline for Hand Washing and Hand Antisepsis in Health-Care Settings (1995), for example, recommends a wash time of 10 to 15 seconds with soap or detergent for routine hand washing for general purposes. The APIC recommends an antimicrobial soap or detergent or alcohol-based rub wash of 10-15 seconds to remove or destroy transient micro-organisms in, for example, nursing and food preparation applications. The APIC further recommends an antimicrobial soap or detergent used with brushing for at least 120 seconds for surgical applications. The US Centers for Disease Control and Prevention (CDC) recommends up to 5 minutes of hand cleaning for surgical applications. Clearly, the length of time spent washing the hands can have a great effect on the eradication of microbes. Thus there is a need for a cleaning formulation that will enable the user to judge how long he has washed his hands in order to comply with the guidelines.

Proper hand washing habits are also important for children. Children in particular need guidance in determining the appropriate amount of time in which hand washing should be performed. This guidance is generally given by parents or other caregivers and, while important, is not omnipresent. In addition to parental guidance, various other mechanisms have been used to encourage children to increase their hand washing times. Soaps have been formulated as foams, for example, to increase the enjoyment children find in hand washing and thus to increase the amount of time children spend by washing.

US 2005/0049157 A1 **(Kimberley-Clark)** suggests a colour change composition that remains stable in a single phase and that contains an indicator that produces an observable colour change after a period of time to show that sufficient cleaning has been done or to indicate the thoroughness of the cleaning. The proposed compositions comprise redox dyes which change their colour when brought into contact with oxygen. This solution, however, is not satisfying since the compositions must be kept strictly away from air contact in order to avoid undesired colour development.

US 2005/ 0112152 A1 discloses encapsulated fragrance materials. The encapsulated fragrance and solvent materials can be further coated with a second coating, preferably a cationic coating, and be added to personal care compositions.

The problem underlying the present invention has therefore been the development of personal care compositions, more particularly, of liquid soaps and hand washing pastes, which give an optical signal to the user after a sufficient degree of cleaning has been reached and avoids the disadvantages of the state of the art.

### Description of the invention

The present invention claims personal care compositions comprising encapsulated dyes.

Surprisingly it has been found that the addition of encapsulated dyes to personal care compositions, for example, - but not limited to - aqueous liquid soaps and hand washing pastes solves the problem outlined above. The capsules are stable enough to survive some mechanical exposure, however break after a certain time releasing the dye which indicates that a sufficient degree of cleaning has been achieved. A special advantage of the composition according to the invention is that it can stay in contact with air without any undesired development of colour.

### Personal care compositions

In a preferred embodiment, personal care compositions according to the present invention represent liquid soaps and hand washing pastes, preferably aqueous liquid soaps and hand washing pastes showing a viscosity according to Brookfield (RVT, spindle 3, 10 rpm) of 300 to 30,000, more preferably 1,000 to 5,000, and most preferably 2,000 to 3,000 mPas. According to a second preferred embodiment, said compositions comprise said micro-encapsulated dyes in amounts sufficient to achieve the desired colouring effect, i.e. in amounts from 0.1 to 80 % b.w., more preferably from 1 to 20 % b.w., and most preferably from 2 to 10 % b.w.

### Micro-encapsulated dyes

In order to achieve the desired colouring effect by breaking the capsules during liquid soap and hand washing on the one side and reducing the amount of micro-capsules in the composition on the other side, another preferred embodiment of the present invention is directed to the use of micro-capsules showing a high content of dyes, particularly of micro-capsules containing 5 to 95 % b.w. and preferably 20 to 50 % b.w. of cosmetically acceptable dyes.

"Microcapsules" are understood to be spherical aggregates with a diameter of about 0.1 to about 5 mm which contain at least one solid or liquid core surrounded by at least one continuous membrane. More precisely, they are finely dispersed liquid or solid phases coated with film-forming polymers, in the production of which the polymers are deposited onto the material to be encapsulated after emulsification and coacervation or interfacial polymerization. In another process, liquid active principles are absorbed in a matrix ("microsponge") and, as micro-particles, may be additionally coated with film-forming polymers. The microscopically small capsules, also known as nanocapsules, can be dried in the same way as powders. Besides single-core microcapsules, there are also multiple-core aggregates, also known as microspheres, which contain two or more cores distributed in the continuous membrane material. In addition, single-core or multiple-core microcapsules may be surrounded by an additional second, third etc. membrane. The membrane may consist of natural, semi-synthetic or synthetic materials. Natural membrane materials are, for example, gum arabic, agar agar, agarose, maltodextrins, alginic acid and salts thereof, for example sodium or calcium alginate, fats and fatty acids, cetyl alcohol, collagen, chitosan, lecithins, gelatin, albumin, shellac, polysaccharides, such as starch or dextran, polypeptides, protein hydrolyzates, sucrose and waxes. Semi-synthetic membrane materials are, inter alia, chemically modified celluloses, more particularly, cellulose esters and ethers, for example cellulose acetate, ethyl cellulose, hydroxypropyl cellulose, hydroxypropyl methyl cellulose and carboxymethyl cellulose, and starch derivatives, more particularly, starch ethers and esters. Synthetic membrane materials are, for example, polymers, such as polyacrylates, polyamides, polyvinyl alcohol or polyvinyl pyrrolidone.

Examples of known microcapsules are the following commercial products (the membrane material is shown in brackets): Hallcrest Microcapsules (gelatin, gum arabic), Coletica Thalaspheres (maritime collagen), Lipotec Millicapseln (alginic acid, agar agar), Induchem Unispheres (lactose, microcrystalline cellulose, hydroxypropylmethyl cellulose), Unicetin C30 (lactose, microcrystalline cellulose, hydroxypropylmethyl cellulose), Kobo Glycospheres (modified starch, fatty acid esters, phospholipids), Softspheres (modified agar agar) and Kuhs Probiol Nanospheres (phospholipids).

The active principles are released from the microcapsules by mechanical, thermal, chemical or enzymatic destruction of the membrane, normally during the use of the preparations containing the microcapsules. From the state of the art is known also a huge number of different processes for the encapsulation of active principles: WO 99/043426**;** WO 01/001928**;** WO 01/001929**;** WO 01/066240**;** WO 01/066241**;** WO 01/098578**;** WO 02/0178859**;** WO 02/0178868**;** WO 02/076607**;** WO 02/076606**;** WO 02/077359**;** WO 02/077360**;** WO 03/022419**;** WO 03/093571**;** WO 03/092664**;** WO 03/092880**;** WO 04/091555**;** WO 04/106621**;** EP 1064911 B1**;** EP 1064912 B1**;** EP 1077060 B1**;** EP 1101527 B1**;** EP 1223243 B1**;** EP 1243318 B1**;** EP 1243320 B1**;** EP 1243323 B1**;** EP 1243324 B1**;** EP 1254983 B1**;** EP 1121542 B1 all filed on behalf of Henkel KGaA, Primacare S.A. or Cognis Iberia, S.L. and herewith incorporated by reference.

Despite the fact that the state of the art cites a huge range of possibilities for the encapsulation of actives, methods according to which a shell is obtained by coacervation, precipitation or polycondensation of anionic and cationic polymers has been quite suitable for the formation of stable capsules. It should be noticed, however, that not all types of micro-capsules fulfil the requirements to solve the underlying problem of the invention in the same way. As a matter of fact, those micro-capsules are preferred, which are sufficiently stable in the presence of surfactants and exhibit also a high stability against any undesired migration of the dye through the shell into the care composition. In addition, although they should break during use, they must be sufficiently stable against mechanical treatment so that they do not break immediately, but after a defined period of time in order to signal that the hands are clean.

Taking this into account, a first type of preferred capsules and a respective process for the encapsulation of active principles according to the present invention is characterised in that it comprises the steps of
(a) preparing a matrix from gel formers, cationic polymers and active principles;
(b) optionally dispersing said matrix in an oil phase; and
(c) treating said dispersed matrix with aqueous solutions of anionic polymers and optionally removing the in phase in the process.

Of course, anionic and cationic polymers in steps (a) and (c) can be exchanged. In another embodiment, the microcapsules according to the present invention can be prepared according to the following two processes:
(a3) preparing a matrix from gel formers, cationic polymers and active principles; and
(b3) dropping said dispersed matrix into aqueous solutions of anionic polymers or ionotropically gellyfying anionic agents.
or
(a4) preparing a matrix from gel formers, anionic polymers and active principles; and
(b4) dropping said dispersed matrix into aqueous solutions of cationic molecules or ionotropically gellifying cationic agents.

### • Gel formers

In the context of the invention, preferred gel formers are substances which are capable of forming gels in aqueous solution at temperatures above 40° C. Typical examples of such gel formers are heteropolysaccharides and proteins. Preferred thermogelling heteropolysaccharides are agaroses which may be present in the form of the agar agar obtainable from red algae, even together with up to 30% by weight of non-gel-forming agaropectins. The principal constituent of agaroses are linear polysaccharides of Galactose and 3,6-anhydro-L-galactose with alternate 1,3- and 1,4-glycosidic bonds. The heteropolysaccharides preferably have a molecular weight of 110,000 to 160,000 and are both odourless and tasteless. Suitable alternatives are pectins, xanthans (including xanthan gum) and mixtures thereof. Other preferred types are those which in 1% by weight aqueous solution still form gels that do not melt below 80° C and solidify again above 40° C. Examples from the group of thermogelling proteins are the various gelatines.

### • Anionic polymers

Salts of alginic acid are preferred for this purpose. The alginic acid is a mixture of carboxyl-containing polysaccharides with the following idealized monomer unit:

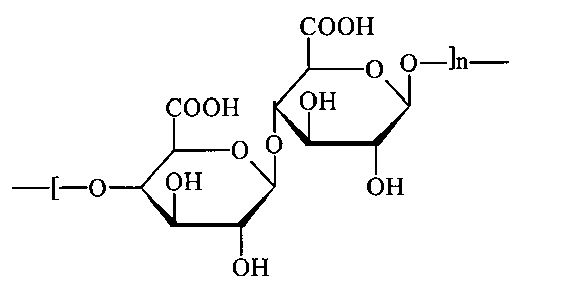

The average molecular weight of the alginic acid or the alginates is in the range from 150,000 to 250,000. The salts of alginic acid and complete and partial neutralization products thereof are in particular understood to be alkali metal salts, preferably sodium alginate ("algin") and the ammonium and alkaline earth metal salts. Mixed alginates, for example sodium/magnesium or sodium/calcium alginates, are particularly preferred. In an alternative embodiment of the invention, however, anionic chitosan derivatives, for example the carboxylation and, above all, succinylation products are also suitable for this purpose.

### • Cationic polymers

Chitosans are biopolymers which belong to the group of hydrocolloids. Chemically, they are partly de-acetylated chitins differing in their molecular weights which contain the following - idealized - monomer unit:

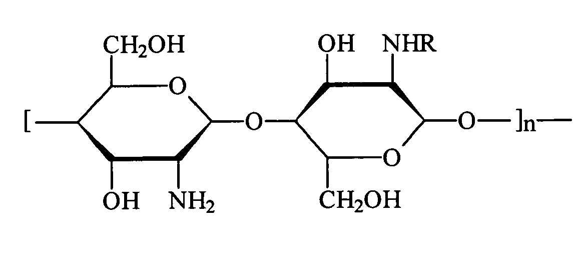

In contrast to most hydrocolloids, which are negatively charged at biological pH values, chitosans are cationic biopolymers under these conditions. The positively charged chitosans are capable of interacting with oppositely charged surfaces and are therefore used in cosmetic hair care and body care products and pharmaceutical preparations. Chitosans are produced from chitin, preferably from the shell residues of crustaceans which are available in large quantities as inexpensive raw materials. In a process described for the first time by Hackmann et al., chitin is normally first de-proteinized by addition of bases, demineralized by addition of mineral acids and, finally, de-acetylated by addition of strong bases, the molecular weights being distributed over a broad spectrum. Preferred types are those which are disclosed in German patent applications DE 4442987 A1 and DE 19537001 A1 (Henkel) and which have an average molecular weight of 10,000 to 500,000 Dalton or 800,000 to 1,200,000 Dalton and/or a Brookfield viscosity (1 % by weight in glycolic acid) below 5,000 mPas, a degree of de-acetylation of 80 to 88 % and an ash content of less than 0.3% by weight. In the interests of better solubility in water, the chitosans are generally used in the form of their salts, preferably as glycolates.

In a preferred embodiment of the invention, an aqueous solution of the gel former of 1 to 10, and preferably 2 to 5 % by weight, preferably agar agar, is normally prepared and heated under reflux. A second aqueous solution containing the cationic polymer, preferably chitosan, in quantities of 0.1 to 2, and preferably 0.25 to 0.5 % by weight and the active principle - the dye - in quantities of 0.1 to 25 and preferably 10 to 20 % by weight, is added in the boiling heat, preferably at 80 to 100°C; this mixture is called the matrix. Accordingly, the charging of the microcapsules with active principles may also comprise 0.1 to 25% by weight, based on the weight of the capsules. If desired, water-insoluble constituents, for example inorganic pigments, may also be added at this stage to adjust viscosity, generally in the form of aqueous or aqueous/alcoholic dispersions. In addition, to emulsify or disperse the active principles, it can be useful to add emulsifiers and/or solubilizers to the matrix. After its preparation from gel former, cationic polymer and active principle, the matrix is optionally very finely dispersed in an oil phase with intensive shearing in order to produce small particles in the subsequent encapsulation process. It has proved to be particularly advantageous in this regard to heat the matrix to temperatures in the range from 40 to 60° C while the oil phase is cooled to 10 to 20° C. The actual encapsulation, i.e. formation of the membrane by contacting the cationic polymer in the matrix with the anionic polymers, takes place in the third step. To this end, it is advisable to wash the matrix - dispersed in the oil phase - with an aqueous solution of the anionic polymer of approx. 0.1 to 3 and preferably 0.25 to 0.5% by weight, preferably the alginate, at a temperature in the range from 40 to 100 and preferably 50 to 60° C and, at the same time, to remove the oil phase if present. The resulting aqueous preparations generally have a microcapsule content of 1 to 20% by weight. In some cases, it can be of advantage for the solution of the polymers to contain other ingredients, for example emulsifiers or preservatives. After filtration, microcapsules with an average diameter of preferably 1 to 3 mm are obtained. It is advisable to sieve the capsules to ensure a uniform size distribution. The microcapsules thus obtained may have any shape within production-related limits, but are preferably substantially spherical. Their average diameter can be controlled by the speed of agitation during the incorporation into the cosmetic oil phase and lies between 0.0001 and 5, preferably 0.001 and 1 mm.

Examples for suitable dyes are any of the substances suitable and approved for cosmetic purposes as listed, for example, in the publication **"**Kosmetische Färbemittel" of the Farbstoffkommission der Deutschen Forschungsgemeinschaft, Verlag Chemie, Weinheim, 1984, pages 81 to 106**.** Examples include cochineal red A (C.I. 16255), patent blue V (C.I. 42051), blue (C.I. 77077), indigotin (C.I. 73015), chlorophyllin (C.I. 75810), quinoline yellow (C.I. 47005), titanium dioxide (C.I. 77891), indanthrene blue RS (C.I. 69800) and madder lake (C.I. 58000). Luminol may also be present as a luminescent dye.

### Industrial application

The personal care compositions according to the present invention may comprise further additives, e.g., surfactants, oil bodies, emulsifiers, superfatting agents, pearlizing waxes, consistency factors, polymers, silicone compounds, waxes, stabilizers, hydrotropes, preservatives and perfume oils in amounts of typically 1 to 50, preferably 5 to 30 % b.w., while the amount of water typically lies between 30 and 70, preferably between 40 and 60 % b.w.

### Surfactants

Other preferred auxiliaries and additives are anionic and/or amphoteric or zwitterionic surfactants. Typical examples of anionic surfactants are soaps, alkyl benzenesulfonates, alkanesulfonates, olefin sulfonates, alkylether sulfonates, glycerol ether sulfonates, methyl ester sulfonates, sulfofatty acids, alkyl sulfates, fatty alcohol ether sulfates, glycerol ether sulfates, fatty acid ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acylamino acids such as, for example, acyl lactylates, acyl tartrates, acyl glutamates and acyl aspartates, alkyl oligoglucoside sulfates, protein fatty acid condensates (particularly wheat-based vegetable products) and alkyl (ether) phosphates. If the anionic surfactants contain polyglycol ether chains, they may have a conventional homologue distribution although they preferably have a narrow-range homologue distribution. Typical examples of amphoteric or zwitterionic surfactants are alkylbetaines, alkylamidobetaines, aminopropionates, aminoglycinates, imidazolinium betaines and sulfobetaines. The surfactants mentioned are all known compounds. Information on their structure and production can be found in relevant synoptic works, cf. for example J. Falbe (ed.), "Surfactants in Consumer Products", Springer Verlag, Berlin, 1987, pages 54 to 124 **or** J. Falbe (ed.), "Katalysatoren, Tenside und Mineralöladditive (Catalysts, Surfactants and Mineral Oil Additives)", Thieme Verlag, Stuttgart, 1978, pages 123-217**.** The percentage content of surfactants in the preparations may be from 0.1 to 10% by weight and is preferably from 0.5 to 5% by weight, based on the preparation.

### Oil bodies

Suitable oil bodies, which form constituents of the O/W emulsions, are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols or esters of branched C₆-C₁₃-carboxylic acids with linear or branched C₆-C₂₂-fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂ fatty acids with branched alcohols, in particular 2-ethylhexanol, esters of C₁₈-C₃₈ alkylhydroxy carboxylic acids with linear or branched C₆-C₂₂-fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C₆-C₁₀ fatty acids, liquid mono-/di-/triglyceride mixtures based on C₆-C₁₈ fatty acids, esters of C₆-C₂₂ fatty alcohols and/or Guerbet alcohols with aromatic carboxylic acids, in particular benzoic acid, esters of C₂-C₁₂ dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂ fatty alcohol carbonates, such as, for example, Dicaprylyl Carbonate (Cetiol^{®} CC), Guerbet carbonates, based on fatty alcohols having 6 to 18, preferably 8 to 10, carbon atoms, esters of benzoic acid with linear and/or branched C₆-C₂₂-alcohols (e.g. Finsolv^{®} TN), linear or branched, symmetrical or asymmetrical dialkyl ethers having 6 to 22 carbon atoms per alkyl group, such as, for example, dicaprylyl ether (Cetiol^{®} OE), ring-opening products of epoxidized fatty acid esters with polyols, silicone oils (cyclomethicones, silicone methicone grades, etc.) and/or aliphatic or naphthenic hydrocarbons, such as, for example, squalane, squalene or dialkylcyclohexanes.

### Emulsifiers

Other surfactants may also be added to the preparations as emulsifiers, including for example:
- products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
- C_{12/18} fatty acid monoesters and diesters of addition products of 1 to 30 mol ethylene oxide onto glycerol;
- glycerol mono- and diesters and sorbitan mono- and diesters of saturated and unsaturated fatty acids containing 6 to 22 carbon atoms and ethylene oxide addition products thereof;
- addition products of 15 to 60 mol ethylene oxide onto castor oil and/or hydrogenated castor oil;
- polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
- addition products of 2 to 15 mol ethylene oxide onto castor oil and/or hydrogenated castor oil;
- partial esters based on linear, branched, unsaturated or saturated C_{6/22} fatty acids, ricinoleic acid and 12-hydroxystearic acid and glycerol, polyglycerol, pentaerythritol, - dipentaerythritol, sugar alcohols (for example sorbitol), alkyl glucosides (for example methyl glucoside, butyl glucoside, lauryl glucoside) and polyglucosides (for example cellulose);
- mono-, di and trialkyl phosphates and mono-, di- and/or tri-PEG-alkyl phosphates and salts thereof;
- wool wax alcohols;
- polysiloxane/polyalkyl polyether copolymers and corresponding derivatives;
- mixed esters of pentaerythritol, fatty acids, citric acid and fatty alcohol and/or mixed esters of C₆₋₂₂ fatty acids, methyl glucose and polyols, preferably glycerol or polyglycerol,
- polyalkylene glycols and
- glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations. The preferred emulsifiers are described in more detail as follows:

### • Partial glycerides

Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.

### • Sorbitan esters

Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.

### • Polyglycerol esters

Typical examples of suitable polyglycerol esters are Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} GI 34), Polyglyceryl-3 Oleate, Diisostearoyl Polyglyceryl-3 Diisostearate (Isolan^{®} PDI), Polyglyceryl-3 Methylglucose Distearate (Tego Care^{®} 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor® GS 32) and Polyglyceryl Polyricinoleate (Admul^{®} WOL 1403), Polyglyceryl Dimerate Isostearate and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

Typical anionic emulsifiers are aliphatic C₁₂₋₂₂ fatty acids, such as palmitic acid, stearic acid or behenic acid, and C₁₂₋₂₂ dicarboxylic acids, such as azelaic acid or sebacic acid.

Other suitable emulsifiers are zwitterionic surfactants. Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopropyl-N,N-dimethyl ammonium glycinates, for example cocoacylaminopropyl dimethyl ammonium glycinate, and 2-alkyl-3-carboxymethyl-3-hydroxyethyl imidazolines containing 8 to 18 carbon atoms in the alkyl or acyl group and cocoacylaminoethyl hydroxyethyl carboxymethyl glycinate. The fatty acid amide derivative known under the CTFA name of *Cocamidopropyl Betaine* is particularly preferred. Ampholytic surfactants are also suitable emulsifiers. Ampholytic surfactants are surface-active compounds which, in addition to a C_{8/18} alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalkylaminopropionate, cocoacylaminoethyl aminopropionate and C_{12/18} acyl sarcosine.

### Superfatting agents

Superfatting agents may be selected from such substances as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers.

### Consistency factors

The consistency factors mainly used are fatty alcohols or hydroxyfatty alcohols containing 12 to 22 and preferably 16 to 18 carbon atoms and also partial glycerides, fatty acids or hydroxyfatty acids. A combination of these substances with alkyl oligoglucosides and/or fatty acid N-methyl glucamides of the same chain length and/or polyglycerol poly-12-hydroxystearates is preferably used.

### Thickening agents

Suitable thickeners are polymeric thickeners, such as Aerosil® types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols® [Goodrich] or Synthalens® [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrow-range fatty alcohol ethoxylates and electrolytes, such as sodium chloride and ammonium chloride.

### Polymers

Suitable cationic polymers are, for example, cationic cellulose derivatives such as, for example, the quaternized hydroxyethyl cellulose obtainable from Amerchol under the name of Polymer JR 400®, cationic starch, copolymers of diallyl ammonium salts and acrylamides, quaternized vinyl pyrrolidone/vinyl imidazole polymers such as, for example, Luviquat® (BASF), condensation products of polyglycols and amines, quaternized collagen polypeptides such as, for example, Lauryldimonium Hydroxypropyl Hydrolyzed Collagen (Lamequat® L, Grfnau), quaternized wheat polypeptides, polyethyleneimine, cationic silicone polymers such as, for example, amodimethicone, copolymers of adipic acid and dimethylaminohydroxypropyl diethylenetriamine (Cartaretine^{®}, Sandoz), copolymers of acrylic acid with dimethyl diallyl ammonium chloride (Merquat^{®} 550, Chemviron), polyaminopolyamides and crosslinked water-soluble polymers thereof, cationic chitin derivatives such as, for example, quaternized chitosan, optionally in microcrystalline distribution, condensation products of dihaloalkyls, for example dibromobutane, with bis-dialkylamines, for example bis-dimethylamino-1,3-propane, cationic guar gum such as, for example, Jaguar^{®}CBS, Jaguar^{®}C-17, Jaguar^{®}C-16 of Celanese, quaternized ammonium salt polymers such as, for example, Mirapol^{®} A-15, Mirapol^{®} AD-1, Mirapol^{®} AZ-1 of Miranol.

Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones.

### Pearlising waxes

Suitable pearlising waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxy-substituted carboxylic acids with fatty alcohols containing 6 to 22 carbon atoms, especially long-chain esters of tartaric acid; fatty compounds, such as for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups and mixtures thereof.

### Silicones

Suitable silicone compounds are, for example, dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Other suitable silicone compounds are simethicones which are mixtures of dimethicones with an average chain length of 200 to 300 dimethylsiloxane units and hydrogenated silicates. A detailed overview of suitable volatile silicones can be found in Todd et al. in Cosm. Toil. 91, 27 (1976**).**

### Waxes

Besides natural oils used, waxes may also be present in the preparations, more particularly natural waxes such as candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as montan ester waxes, sasol waxes, hydrogenated jojoba waxes and synthetic waxes such as polyalkylene waxes and polyethylene glycol waxes.

### Stabilizers

Metal salts of fatty acids, for example magnesium, aluminium and/or zinc stearate or ricinoleate may be used as stabilizers.

### Hydrotropes

In addition, hydrotropes, for example ethanol, isopropyl alcohol or polyols, may be used to improve flow behaviour. Suitable polyols preferably contain 2 to 15 carbon atoms and at least two hydroxyl groups. The polyols may contain other functional groups, more particularly amino groups, or may be modified with nitrogen. Typical examples are
- glycerol;
- alkylene glycols such as, for example, ethylene glycol, diethylene glycol, propylene glycol, butylene glycol, hexylene glycol and polyethylene glycols with an average molecular weight of 100 to 1000 Dalton;
- technical oligoglycerol mixtures with a degree of self-condensation of 1.5 to 10, such as technical diglycerol mixtures with a diglycerol content of 40 to 50% by weight;
- methylol compounds such as, in particular, trimethylol ethane, trimethylol propane, trimethylol butane, pentaerythritol and dipentaerythritol;
- lower alkyl glucosides, particularly those containing 1 to 8 carbon atoms in the alkyl group, for example methyl and butyl glucoside;
- sugar alcohols containing 5 to 12 carbon atoms, for example sorbitol or mannitol,
- sugars containing 5 to 12 carbon atoms, for example glucose or sucrose;
- amino sugars, for example glucamine;
- dialcoholamines, such as diethanolamine or 2-aminopropane-1,3-diol.

### Preservatives

Suitable preservatives are, for example, phenoxyethanol, formaldehyde solution, parabens, pentanediol or sorbic acid and the other classes of compounds listed in Appendix 6, Parts A and B of the Kosmetikverordnung ("Cosmetics Directive").

### Perfume oils

Suitable perfume oils are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, α-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

### Examples

### Example 1

### Preparation of Dye Primasponge ^{®} Blue Capsules

A first pre-mixture "A" was prepared by dissolving 1 g agar-agar in 43 ml of boiling water. For pre-mixture "B" 0.5 g Phenonip^{®} was dissolved in 15 ml of a 2 % b.w. aqueous solution of sodium alginate. A third pre-mixture "C" was obtained by dispersing 20 g of a blue dye (CI 77007) in 30 g of glycerol. Finally, a pre-mixture "D" was prepared by mixing 5 g shea butter (Cetiol^{®} SB-45) with 0.5 g of a non-ionic emulsifier (Tween^{®}-20). Subsequently, pre-mixtures B, C and D were added stepwise to pre-mixture A. The mixture thus obtained was kept at about 70 °C, that means a temperature above the gelification point of the agar, and was slowly dispersed under agitation into 250 ml of a cosmetic oil (Cetiol^{®} 868) having a temperature of about 20 °C. When dropped into the cold oil, the droplets gelled and blue spheres were formed, their diameter being controlled by the speed of agitation. Afterwards, the capsules were removed by filtration, washed with an aqueous solution comprising 1 % Tween-20 and 0.1 b.w. % of chitosan glycolate. Finally, the capsules were dispersed in an external phase comprising 10 % propylene glycol, 0.4 % b.w. Phenonip, and 0.4 % b.w. phenoxy ethanol.

### Example 2

### Preparation of a liquid soap with encapsulated dyes

**Table 1 Liquid soap**

| **Phase** | **Components** | **INCI** | **% [b.w.]** |
|---|---|---|---|
| I | Water | | 30.0 |
| | Carbopol^{®} Aqua SF 1 | Acrylates copolymer | 8.0 |
| | NaOH dil. 10% | | appr. 3.4 |
| II | Water | | up to 100 |
| | Glycerine | Glycerol | 1.0 |
| | Hexylene Glycol | Hexylene glycol | 1.0 |
| | Plantapon^{®} 611 C | Sodium laureth sulfate (and) Cocamidopropyl Betaine (and) Coco Glucoside | 20.0 |
| | Irgasan^{®} DP 300 | Triclosan | 0.3 |
| III | Cetiol^{®} LDO | Dicaprylyl Ether (and) Lauryl Alcohol | 0.2 |
| IV | Dye Primasponge^{®} Blue | | 4.0 |

The ingredients of Phase I were mixed until transparency was reached, and the pH value adjusted to 6.7 to 7.2. The ingredients of Phase II ware stirred until homogeneity was reached and also adjusted to a pH of 6.7 - 7.2. Subsequently, Phase II was given over Phase I avoiding any incorporation of air. Then the remaining ingredients were added to the composition, while the *Primasponges*^{®} were added as the last component under gentle stirring. The paste showed a viscosity according to Brookfield (RVT, spd 3,10 rpm) of 2,700 mPas.

## Claims

1. Personal care compositions comprising encapsulated dyes.

2. Compositions according to claim 1, **characterised in that** they represent liquid soaps or hand washing pastes.

3. Compositions according to claims 1 and/or 2, **characterised in that** they represent aqueous liquid soaps or hand washing pastes showing a viscosity according to Brookfield (RVT, spindle 3, 10 rpm) of 300 to 30,000 mPas.

4. Compositions according to any of claims 1 to 3, **characterised in that** they comprise micro-encapsulated dyes in amounts of 0.1 to 80 % b.w.

5. Compositions according to any of claims 1 to 4, **characterized in that** they comprise micro-capsules containing 5 to 95 % b.w. of cosmetically acceptable dyes.

6. Compositions according to any of claims 1 to 5, **characterised in that** they comprise micro-capsules obtained by the steps of
(a1) preparing a matrix from gel formers, cationic polymers and active principles;
(b1) optionally dispersing said matrix in an oil phase; and
(c1) treating said dispersed matrix with aqueous solutions of anionic polymers, and optionally removing the oil phase in the process
or
(a2) preparing a matrix from gel formers, anionic polymers and active principles;
(b2) optionally dispersing said matrix in an oil phase; and
(c2) treating said dispersed matrix with aqueous solutions of cationic polymers, and optionally removing the oil phase in the process
or
(a3) preparing a matrix from gel formers, cationic polymers and active principles; and
(b3) dropping said dispersed matrix into aqueous solutions of anionic polymers or ionotropically gelifying anionic agents
or
(a4) preparing a matrix from gel formers, anionic polymers and active principles; and
(b4) dropping said dispersed matrix into aqueous solutions of cationic molecules or ionotropically gelifying cationic agents.

7. Compositions according to any of the claims 1 to 6, **characterised in that** they comprise micro-capsules having an average diameter of 0.0001 to 5 mm.
